# EUROPEAN PATENT APPLICATION

(11) **EP 3 072 552 A1**
(43) Date of publication of application: **28.09.2016**
(21) Application number: 16160681.9
(22) Date of filing: 16.03.2016
(51) Int. Cl.: A61M 25/04

(54) **MEDICAL DEVICE WITH BIASING MEMBER**

(30) Priority: 19.03.2015 US 201562135383 P
(71) Applicant: Cook Medical Technologies LLC, Bloomington, IN 47404 (US)
(72) Inventor: PENDLETON, Steven L., Spencer, IN 47460 (US); KRIEGER, Joshua F., Bloomington, IN 47404 (US)
(74) Representative: Garratt, Peter Douglas

(57) **Abstract**

A tubular medical device, such as an elongate catheter is provided. The catheter includes an anchor mechanism with two or more arms biased toward an expanded position and can be urged toward a narrower insertion position. Each of the two or more arms are fixed to the tube proximate a nonexpandable distal tip of the tube and to the central portion of the tube. A biasing member is disposed in conjunction with the anchor mechanism, the biasing member is connected to the tube at the central portion proximate to a position where the two or more arms connect to the tube and is also connected to the tube proximate to the distal tip. The biasing member urges the two or more arms toward the expanded position.

## Description

### CROSS-REFERENCE TO RELATED APPLICATIONS

This application claims priority from United States Provisional Application No. 62/135,383 filed on March 19, 2015.

### TECHNICAL FIELD

The present disclosure relates to catheters and methods of using the same, and more specifically, to a catheter for draining a body cavity.

### BACKGROUND

Catheters may be used for many purposes including draining fluids, such as urine or bile, from a body cavity. A drainage catheter may be inserted into the body through an orifice or the skin until it reaches a cavity in the body where fluid exists. Drainage catheters commonly have a proximal end portion and a distal end portion with an anchor at the distal end portion to secure the drainage catheter in the desired cavity in the body. These anchors can be referred to as a Malecot. Malecots are commonly used as a bladder or kidney anchoring mechanisms in urinary drainage catheters and nephrostomy tubes.

This type of anchor is commonly made up of at least two wings or arms, which may be continuous with the rest of the catheter and may be made from the same material. These wings or arms are typically biased toward an expanded position, so that when no force is applied to the wings or arms, they can potentially hold the drainage catheter in place in a body cavity. While drainage catheters can be made of several materials, often, a thick-walled material must be used in order for the wings or arms to possess enough shape memory to function as an effective anchor.

### SUMMARY

A first embodiment of the disclosure is provided. The embodiment includes a drainage catheter with a Malecot-type anchoring mechanism and a spring that is incorporated into the Malecot-type anchoring mechanism. The drainage catheter may be a long narrow tube or shaft having an outer diameter and a smaller inner diameter. The drainage catheter may also have an inner lumen adapted to allow fluid to flow therethrough. The drainage catheter may also have a proximal end portion and a distal end portion. The drainage catheter may also be used in nephrostomy tubes, biliary drainage catheters, and urinary drainage catheters.

At the distal end portion there may be a Malecot-type anchoring mechanism having a top portion and a bottom portion. The Malecot-type anchoring mechanism may also consist of a plurality of expandable wings or arms which are biased toward a first radially expanded open position and are moveable toward a second radially compressed insertion position. The Malecot-type anchoring mechanism may also comprise a spring that is connected to the top portion and the bottom portion of the Malecot-type anchoring mechanism.

The spring may be biased toward a compressed position which may put an inward force on the Malecot-type anchoring mechanism so that the top portion and the bottom portion are urged toward one another and the plurality of expandable wings or arms are further urged in the first radially expanded open position. The spring may also be urged toward an expanded position by an external force to allow the Malecot-type anchoring mechanism to be moved toward a radially compressed insertion position. After the external force is removed, the spring may be formed from a superelastic material that will allow it to return to its original compressed position. Using a spring attached to the Malecot anchoring device, the drainage catheter can be made of a thinner walled catheter material and achieve the same Malecot shape memory as a thicker walled catheter material without a spring.

Advantages of the disclosed devices will become more apparent to those skilled in the art from the following description of embodiments that have been shown and described by way of illustration. As will be realized, other and different embodiments are contemplated, and the disclosed details are capable of modification in various respects. Accordingly, the drawings and description are to be regarded as illustrative in nature and not as restrictive.

### BRIEF DESCRIPTION OF THE DRAWINGS

FIG. 1 is a perspective view of a drainage catheter with the plurality of arms in a radially expanded open position.
FIG. 2 is a close up perspective view of a Malecot-type anchoring mechanism in a radially expanded open position.
FIG. 3 is a view of the inside of a tube or shaft of drainage catheter illustrating an inner and outer diameter.
FIG. 4 is a perspective view of a drainage catheter with the plurality of arms in a radially compressed insertion position.
FIG. 5 is another perspective view of a drainage catheter with the plurality of arms in a radially compressed insertion position.

### DETAILED DESCRIPTION

Drainage catheters are designed to remove undesired fluids from body cavities such as the kidney and bladder. The drainage catheters may be a long narrow tube or shaft having an outer diameter and a smaller inner diameter. An anchoring mechanism may be provided at or proximate to the distal end portion of the tube. The anchoring mechanism may be a malecot having a plurality of expandable wings or arms that are biased toward an expanded position. The plurality of expandable wings or arms may be from an elastic material, such as a superelastic material that allows the material to return to its original shape after an external force has caused a deformation in the plurality of expandable wings or arms is removed.

Sometimes, certain materials fail to return to their original shape after removal of an external force if the elasticity of the material is not strong enough initially or if the material has weakened over time. If the anchoring mechanism does not have a sufficient elasticity, it can often fail to effectively anchor the drainage catheter in place in a body cavity. If a material is too thick, it can cause complications with insertion and removal of the drainage catheter, which can also create a dangerous or uncomfortable situation for a patient.

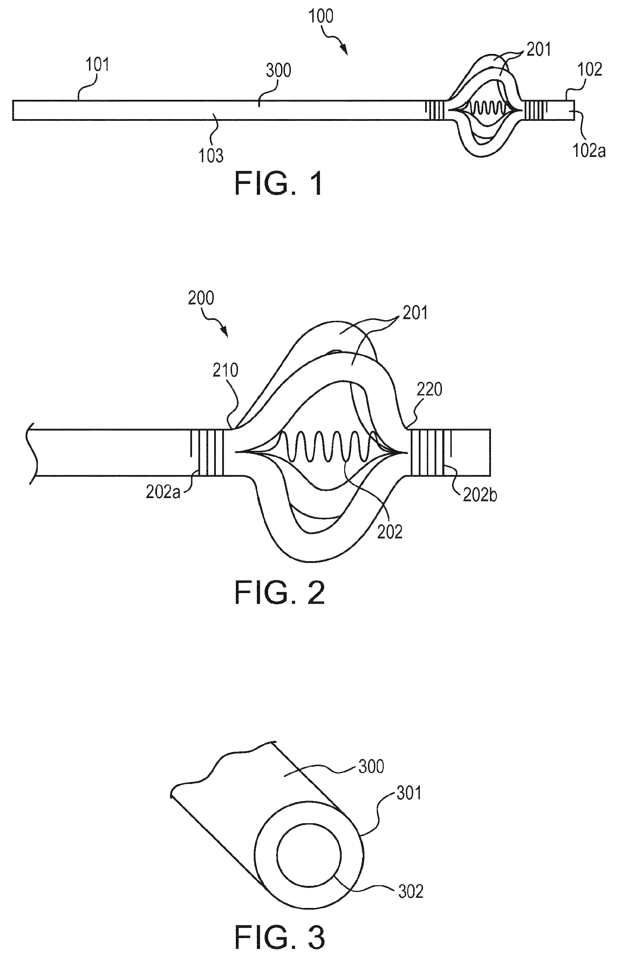

One embodiment of a typical drainage catheter 100 for use in draining fluids from a body cavity is depicted in FIG. 1. The drainage catheter 100 may include a long tube or shaft 300, a proximal end portion 101, a distal end portion 102 (with a distal tip 102a), with a central portion 103 disposed therebetween. A malecot-type anchoring mechanism 200 may be provided at the distal end portion 102 that may include a plurality of malecot arms 201. The drainage catheter 100 may be adapted for insertion into a body with manipulation of the malecot-type anchoring mechanism 200. The malecot arms 201 may each have a first end that extends from a portion of the tube 100 proximate to the distal tip 102a and a second end that extends from the central portion 103 of the tube. In some embodiments, the distal tip 102a of the tube may be nonexpandable, i.e. the diameter of the distal tip 102a remains substantially constant when malecot arms 201 are urged toward the insertion configuration (discussed above) and are allowed to return to the normal biased expanded configuration.

The malecot type anchoring mechanism 200 may include a plurality of arms 201, such as 2, 4, 6, 8, or any number of arms that may be appropriate for the necessary anchoring of the tube 100 in place (as will be readily understood or calculated by one of ordinary skill in the art with a thorough review of this specification and the clinical use of the device). The arms 201 may be formed to be biased into an extended position, and may be capable of being urged into a smaller insertion position. In some embodiments, the plurality of arms 201 collectively form an outer diameter that is significantly larger than the outer diameter of the central portion 103 of the tube 300 when the arms 201 are in the expanded position. For example, the outer diameter of the plurality of arms 201 in the expanded configuration may be referenced as a ratio of the diameter of the central portion of the tube 300, and may be such ratios as 2:1, 3:1, 4:1, 5:1, 6:1, 8:1, and 10:1 (including all ratios in this range that are not whole numbers, e.g. 4.5:1) or other ratios that may be clinically suitable. One of ordinary skill in the art, after a thorough review of this specification will contemplate that other ratios may be possible and are within the scope of this disclosure.

Turning now to FIG. 2, a view of the malecot-type anchoring mechanism 200 is provided. The Malecot-type anchoring mechanism 200 may include a bottom portion 210, a top portion 220, a plurality of expandable wings or arms 201, and a spring 202. In some embodiments, the spring 202 may be made of wire, which may be made from a non-corrosive material, and in some embodiments may be made from a superelastic material, such as nitinol. Opposite end portions 202b, 202a of the spring 202 may be connected to the Malecot-type anchoring mechanism 200 at each of the top portion 220 and the bottom portion 210, respectively. The connection of the spring 202 to the top portion 220 and the bottom portion 210 of the Malecot-type anchoring mechanism 200 may be made by wrapping the respective end portion 202b, 202a of the spring 202 around the tube or shaft 300 of the drainage catheter 100 at the top portion 220 and the bottom portion 210 of the Malecot-type anchoring mechanism 200.

In one embodiment of the disclosure, the plurality of expandable wings or arms 201 may be biased toward a first radially expanded open position and may be moveable toward a second radially compressed insertion position with the arms 201 collectively compressed into an outer diameter that is less than the outer diameter when the arms 201 are in the expanded position. In some embodiments, when the arms 201 are compressed into the insertion position, the arms 201 collectively form the same outer diameter as the outer diameter of the central portion 103 of the tube 300, or in other embodiments, the outer diameter of the arms 201 may be within 5, 10, 15, 20, or 25 percent of the outer diameter of the central portion 103.

In some embodiments, the plurality of expandable wings or arms 201 may be elastic and may be trained or configured to be urged toward the radially expanded open position due to the construction of the arms 201. In some embodiments, the arms may be formed from polymers or plastics that may be formed with a biasing force toward the radially expanded open position. In some embodiments, the arms 201 may include a superelastic wire or wires that are trained to extend toward the radially expanded open position, such as after an external force that urged the arms 201 toward the insertion position is removed.

The spring 202 also may be formed from an elastic material, such as superelastic material, such as nitinol including various alloys of nitinol. By connecting to the Malecot-type anchoring mechanism 200 at the top portion 220 and the bottom portion 210 of the spring, the spring 202 may be urged in compression (i.e. the spring 201 is biased to compress along its length), urging the top portion 220 and the bottom portion 210 toward one another. This biasing force of the spring 202 may further assist the plurality of expandable wings or arms 201 in returning toward their original positions after an external force that urged the malecot arms 201 to the insertion position is removed.

As best shown in FIG. 3, which depicts the inside of the tube or shaft 300 of the drainage catheter 100, the tube or shaft 300 may have an outer diameter 301 and an inner diameter 302 that is less than the diameter of the outer diameter 301. A greater wall thickness, which provides for a greater distance between the outer diameter 301 and the inner diameter 302 may create a greater memory strength of the material out of which the tube or shaft 300 is made. In some embodiments, the plurality of expandable wings or arms 201 of the Malecot-type anchoring device 200 may be made of the same material as the tube or shaft 300 of the drainage catheter 100. In other words, the expandable wings or arms 201 may have the same thickness as the remaining portions of the tube or shaft 300. Therefore, an increase in tube thickness may create greater memory strength in the plurality of expandable wings or arms 201 of the Malecot-type anchoring mechanism 200.

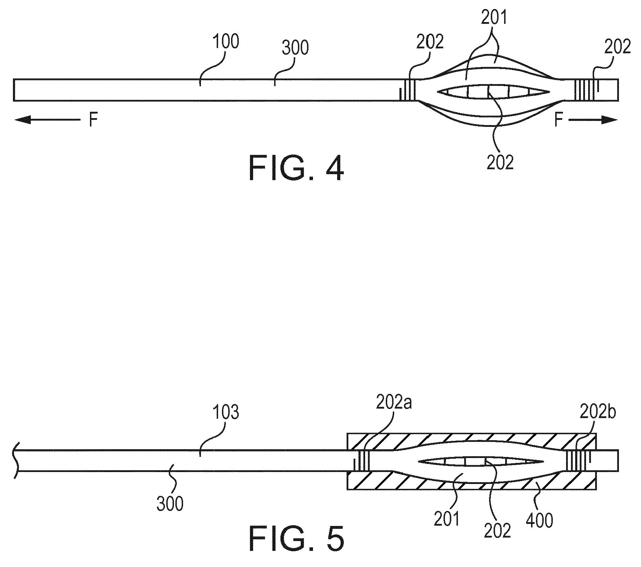

One embodiment of a typical drainage catheter 100 for use in draining fluids from a body cavity in an insertion position is depicted in FIG. 4. This illustrates the plurality of expandable wings or arms 201 of the Malecot-type anchoring mechanism 200 being urged toward the second radially compressed insertion position by an external tension force F being applied to the tube 100. As depicted, the external force F may urge the plurality of expandable wings or arms 201 of the Malecot-type anchoring mechanism 200 toward the second radially compressed insertion position from the first radially expanded open position. In other embodiments shown in FIG. 5, the malecot arms 201 may each be urged toward the compressed insertion position by locally compressing the arms 201, such as with an external component disposed around the arms, the arms 201 being threaded through an external sheath 400, and the hoop strength of the sheath 400 imparts a force that urges or compresses each of the arms 201 inwardly toward the insertion position.

When the external force F is removed (or in other embodiments when the inward force upon the arms 201 is removed), the plurality of wings or arms 201 may return to or toward the first radially expanded open position based upon the outer biasing force of the arms 201 as further urged by the spring 202.

While the preferred embodiments of the disclosure have been described, it should be understood that the disclosure is not so limited and modifications may be made without departing from the invention. The scope of the invention is defined by the appended claims, and all devices that come within the meaning of the claims, either literally or by equivalence, are intended to be embraced therein.

## Claims

1. A medical device comprising:
a tube having a distal end portion, a central portion, and a proximal end portion with a lumen disposed therethrough, the central portion and proximal end portions each having a first outer diameter and an inner diameter;
wherein the distal end portion comprises an anchor mechanism, the anchor mechanism having two or more arms biased toward an expanded position such that when in the expanded position the two or more arms collectively establish a second outer diameter that is larger than the first outer diameter;
wherein each of the two or more arms have a distal end portion that is fixed to the tube proximate a nonexpandable distal tip of the tube, and each of the two or more arms have a proximal end portion that is fixed to the central portion of the tube;
wherein the two or more arms are collapsible into a straightened configuration where the collective outer diameter of the two or more arms is substantially the same as the first outer diameter;
a biasing member disposed in conjunction with the anchor mechanism, the biasing member is connected to the tube at the central portion proximate to a position where the two or more arms connect to the tube and is also connected to the tube proximate to the distal tip;
wherein the biasing member urges the two or more arms toward the expanded position.

2. The medical device of claim 1, wherein the biasing member is a spring, preferably a coil spring.

3. The medical device of claim 2, wherein the spring is made of a non-corrosive material.

4. The medical device of either of claims 2 or 3, wherein the spring is made a superelastic material.

5. The medical device of any one of the preceding claims, wherein a ratio of the diameter of the two or more arms in the expanded configuration to the diameter of the central portion is within a range of 2:1 to 10:1.

6. The medical device of any one of the preceding claims, wherein the tube is adapted to receive a drainage lumen.

7. The medical device of any one of the preceding claims, wherein the tube is adapted for use as a drainage catheter.

8. The medical device of any one of the preceding claims, wherein the tube is adapted for use as a nephrostomy tube.

9. The medical device of any one of the preceding claims, wherein the biasing member operates in compression.

10. The medical device of any one of the preceding claims, wherein the biasing member extends along the longitudinal axis of the tube.
